# EUROPEAN PATENT APPLICATION

(11) **EP 3 616 690 A1**
(43) Date of publication of application: **04.03.2020**
(21) Application number: 19202570.8
(22) Date of filing: 03.10.2012
(51) Int. Cl.: A61K 31/00, A61K 31/4995, A61P 37/06, A61P 37/08, A61P 11/06, G01N 33/50, G01N 33/68

(54) **METHODS AND PHARMACEUTICAL COMPOSITIONS FOR THE TREATMENT OF TH2 MEDIATED DISEASES**

(30) Priority: 03.10.2011 EP 11306272; 04.10.2011 US 201161542860 P
(62) Divisional of application: 12768811.7
(71) Applicant: INSERM (Institut National de la Santé et de la Recherche Médicale), 75013 Paris (FR); Institut Curie, 75005 Paris (FR); CENTRE NATIONAL DE LA RECHERCHE SCIENTIFIQUE -CNRS-, 75016 Paris (FR)
(72) Inventor: AMIGORENA, Sebastian, 75248 PARIS CEDEX 5 (FR); ALLAN, Rhys, 75248 PARIS CEDEX 5 (FR); SCHREIBER, Heidi, 75248 PARIS CEDEX 05 (FR); ZUEVA, Elina, 75248 PARIS CEDEX 05 (FR); ALMOUZNI, Geneviève, 75248 PARIS CEDEX 5 (FR)
(74) Representative: Plasseraud IP

(57) **Abstract**

The present invention relates to methods and pharmaceutical composition for the treatment of T-helper type 2 (Th2)-mediated diseases. More particularly, the present invention relates to an inhibitor of the Suv39h1-HP1α silencing pathway for use in the treatment of a T-helper type 2 (Th2)-mediated disease, in particular allergic asthma.

## Description

### FIELD OF THE INVENTION:

The present invention relates to methods and pharmaceutical composition for the treatment of T-helper type 2 (Th2)-mediated diseases.

### BACKGROUND OF THE INVENTION:

After antigen encounter, CD4+ helper T (Th) cell differentiation follows distinct developmental programs that generate different types of effector T lymphocytes, including Th1, Th2, Th17 or Treg. Each subtype is characterized by specific expression of lineage-instructive transcription factors and signature cytokines. Recently, the stability and plasticity of Th phenotypes has been recognized as critical to immune responses. Differentiation of Th1 and Th2 cells from a common precursor occurs by cross-antagonism between the master-regulators T-bet and GATA-3, respectively, and leads to a mutually exclusive expression of cytokines genes. Th1 cells produce IFNγ, which is important for clearance of intracellular pathogens, whereas Th2 cells produce IL-4, IL-5, IL-10 and IL-13 and are critical for humoral immunity and clearance of extracellular pathogens. These Th2 cytokines play an important role in the pathophysiology of allergic diseases including asthma.

Asthma is a chronic disease that involves inflammation of the pulmonary airways and bronchial hyper-responsiveness leading to reversible obstruction of the lower airways. In a diagnostic context bronchial hyper-responsiveness is evidenced by decreased bronchial airflow following exposure to methacholine or histamine. Natural triggers that provoke airway obstruction include respiratory allergens, cold air, exercise, viral upper respiratory infection, and cigarette smoke. Bronchial provocation with allergen induces a prompt early phase immunoglobulin E (IgE)-mediated decrease in bronchial airflow followed in many patients by a late-phase IgE-mediated reaction with a decrease in bronchial airflow for 4-8 hours. Asthmatic airways display lung hyperinflation, smooth muscle hypertrophy, fibrosis in the lamina reticularis, mucosal edema, epithelial cell sloughing, cilia cell disruption, and mucus gland hypersecretion. Microscopically, asthma is characterized by the presence of increased numbers of eosinophils, mast cells, neutrophils, lymphocytes, and plasma cells in the bronchial tissues, bronchial secretions, and mucus. Activated CD4 T-lymphocytes that produce a Th2 pattern of cytokines appear to be central to the initiation, development and maintenance of the disease phenotype. For example, the cytokines produced by these cells (including IL-4, IL-5, IL-9 and IL-13) regulate infiltration and mediator release by inflammatory cells and allergen specific antibody isotype switching from IgM to IgE. The activity of non-hemopoietic cells, for example mucus hypersecretion by goblet cells, is also regulated by Th2 cytokines.

Accordingly, methods for modulating Th2 immune response are highly desirable for the treatment of Th2-mediated diseases.

As Th1 and Th2 phenotypes are heritable through cellular divisions, it has been proposed that epigenetic modifications regulate T cell differentiation. Interestingly, correlations were observed between levels of several histone-H3 and H4 modifications with activity or silencing of cytokine genes in committed Th1 and Th2 cells. Moreover, the combination of active (H3K4me3) and inactive (H3K27me3) marks in the loci of transcription factors was proposed to shape their transcriptional potential. However, the actual pathways underlying the establishment of epigenetic marks have not been directly manipulated to demonstrate their implication in T cell differentiation.

### SUMMARY OF THE INVENTION:

The present invention relates to an inhibitor of the Suv39h1-HP1α silencing pathway for use in the treatment of a T-helper type 2 (Th2)-mediated disease, in particular allergic asthma.

### DETAILED DESCRIPTION OF THE INVENTION:

The inventors identify an epigenetic pathway that maintains Th2 cell commitment. In Th2 cells the regulatory regions of the silenced Th1 genes (Ifng and Tbx21) bear high levels of the repressive histone mark H3K9me3 and low levels of the active H3K9ac mark. The balance between methylation and acetylation at this position was impaired in Th2 cells deficient for the H3K9-histone methyltransferase Suv39h1. Nevertheless, these cells still presented a completely normal Th2 profile. Only upon re-culture in Th1 conditions did Suv39h1-deficient Th2 cells, in contrast to wild type, re-express the Ifng and Tbx21 genes. Stable Th1 gene silencing in Th2 cells required the expression of the H3K9me3-binding heterochromatin protein 1-α, which was recruited to the Th1 gene promoters in a Suv39h1-dependent manner. In a murine model of Th2 allergic asthma, the loss of Suv39h1 also resulted in skewing towards a Th1 response and decreased lung pathology. The inventors conclude that the Suv39h1-HP1α silencing pathway epigenetically locks away Th1 genes to maintain the fidelity of Th2 cell lineage both in vitro and in vivo and thus inhibition of Suv39h1 may be particularly suitable for the treatment of Th2-mediated diseases.

Accordingly the present invention relates to an inhibitor of the Suv39h1-HP1α silencing pathway for use in the treatment of a T-helper type 2 (Th2)-mediated disease.

As used herein the term "HP1α" has its general meaning in the art and refers to the heterochroatin protein 1 alpha having a single N-terminal chromodomain which can bind to histone proteins via methylated lysine residues, and a C-terminal chromo shadow-domain (CSD) which is responsible for the homodimerization and interaction with a number of chromatin-associated nonhistone proteins residues (Saunders WS, Chue C, Goebl M, Craig C, Clark RF, Powers JA, Eissenberg JC, Elgin SC, Rothfield NF, Earnshaw WC. Molecular cloning of a human homologue of Drosophila heterochromatin protein HP1 using anti-centromere autoantibodies with anti-chromo specificity. J Cell Sci. 1993 Feb;104 (Pt 2):573-82. Said protein is also known as chromobox protein homolog 5, HP1Hs alpha, antigen p25, HP1 alpha homolog, heterochromatin protein 1-alpha, heterochromatin protein 1 homolog alpha, and chromobox homolog 5 (HP1 alpha homolog, Drosophila).

As used herein the term "Suv39h1" or "H3K9-histone methyltransferase Suv39h1" has its general meaning in the art and refers to the histone methyltransferase "suppressor of variegation 3-9 homolog 1 (Drosophila)" that methylates Lys-9 of histone H3 (Aagaard L, Laible G, Selenko P, Schmid M, Dorn R, Schotta G, Kuhfittig S, Wolf A, Lebersorger A, Singh PB, Reuter G, Jenuwein T (Jun 1999). "Functional mammalian homologues of the Drosophila PEV-modifier Su(var)3-9 encode centromere-associated proteins which complex with the heterochromatin component M31". EMBO J 18 (7): 1923-38.). Said histone methyltransferase is also known as MG44, KMT1A, SUV39H, histone-lysine N-methyltransferase SUV39H1, H3-K9-HMTase 1, OTTHUMP00000024298, Su(var)3-9 homolog 1, lysine N-methyltransferase 1A, histone H3-K9 methyltransferase 1, position-effect variegation 3-9 homolog, histone-lysine N-methyltransferase, or H3 lysine-9 specific 1. The term encompasses all orthologs of Suv39h1 such as SU(VAR)3-9.

As used herein, the term "Suv39h1-HP1α silencing pathway" refers to the pathway first described by Bannister, A. J. et al. Selective recognition of methylated lysine 9 on histone H3 by the HP1 chromo domain. Nature 410, 120-124, doi:10.1038/35065138. In the context of the invention, the Suv39h1 trimethylates H3K9 leading to the recruitment of HP1α, which either sterically inhibits the binding of transcriptional machinery or attracts diverse chromatin modifiers, allowing maintenance and propagation of heterochromatin and finally locks expression of the expression of Th1 genes, such as genes encoding for cytokine IFNγ and master regulator Tbet. In particular, the inventors show that the critical part of repressive H3K9 trimethylation within the promoter and several enhancers of Ifng locus is established by Suv39h1. The lost of this trimethylation in Suv39h1-deficient Th2 cells leads to the accumulation of H3K9 acetylation (active mark) and the lost of HP1α. Moreover, in the absence of Suv39h1, H3K9ac accumulates not only in the regulatory elements of Ifng but in the promoter of Tbx21 (coding Tbet) as well. This happens potentially due to the lack of histone deacetylating enzymes (HDACs), which are normally recruited by Suv39h1 to the site of repression. Increase in acetylation impedes the binding of HP1α to the remaining H3K9me3. Thus, the lack of both enzymatic activity of Suv39h1 and the recruitment of HDAC lead to the lost of HP1α binding to Th1 genes. This results in the unlocking of stably silenced Ifng and tbx21 thereby compromising the fidelity of Th2 phenotype.

According to the invention, the inhibitor of the Suv39h1-HP1α silencing pathway is selected from the group consisting of inhibitors of H3K9-histone methyltransferase Suv39h1; inhibitors of H3K9-histone methyltransferase Suv39h1 gene expression, inhibitors of HP1α gene expression and inhibitors of the binding of H3K9me3 to HP1α.

The term "inhibitor of H3K9-histone methyltransferase Suv39h1" refers to any compound natural or not having the ability of inhibiting the methylation of Lys-9 of histone H3 by H3K9-histone methyltransferase Suv39h1.

The inhibiting activity of a compound may be determined using various methods as described in Greiner D. Et al. Nat Chem Biol. 2005 Aug;1(3):143-5 or Eskeland, R. et al. Biochemistry 43, 3740-3749 (2004).

In the context of the present invention, inhibitors of H3K9-histone methyltransferase Suv39h1 are preferably selective for H3K9-histone methyltransferase Suv39h1 as compared with other histone methyltransferases such Suv39h2. By "selective" it is meant that the affinity of the inhibitor is at least 10-fold, preferably 25-fold, more preferably 100-fold, still preferably 500-fold higher than the affinity for other histone methyltransferases.

Typically, the inhibitor of H3K9-histone methyltransferase Suv39h1 is a small organic molecule. The term "small organic molecule" refers to a molecule of a size comparable to those organic molecules generally used in pharmaceuticals. The term excludes biological macromolecules (e. g., proteins, nucleic acids, etc.). Preferred small organic molecules range in size up to about 5000 Da, more preferably up to 2000 Da, and most preferably up to about 1000 Da.

In a particular embodiment, the inhibitor of H3K9-histone methyltransferase Suv39h1 is chaetocin (CAS 28097-03-2) as described by Greiner D, Bonaldi T, Eskeland R, Roemer E, Imhof A. Identification of a specific inhibitor of the histone methyltransferase SU(VAR)3-9. Nat Chem Biol. 2005 Aug;1(3):143-5. Epub 2005 Jul 17.; Weber, H. P., et al., The molecular structure and absolute configuration of chaetocin. Acta Cryst., B28, 2945-2951 (1972) ; Udagawa, S., et al., The production of chaetoglobosins, sterigmatocystin, O-methylsterigmatocystin, and chaetocin by Chaetomium spp. and related fungi. Can. J. microbiol., 25, 170-177 (1979).; Gardiner, D. M., et al., The epipolythiodioxopiperazine (ETP) class of fungal toxins: distribution, mode of action, functions and biosynthesis. Microbiol., 151, 1021-1032 (2005). For example, chaetocin is commercially available from Sigma Aldrich.

In another embodiment, the inhibitor of H3K9-histone methyltransferase Suv39h1 is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

Inhibitors of expression for use in the present invention may be based on anti-sense oligonucleotide constructs. Anti-sense oligonucleotides, including anti-sense RNA molecules and anti-sense DNA molecules, would act to directly block the translation of H3K9-histone methyltransferase Suv39h1 or HP1α mRNA by binding thereto and thus preventing protein translation or increasing mRNA degradation, thus decreasing the level of H3K9-histone methyltransferase Suv39h1 or HP1α, and thus activity, in a cell. For example, antisense oligonucleotides of at least about 15 bases and complementary to unique regions of the mRNA transcript sequence encoding H3K9-histone methyltransferase Suv39h1 or HP1α can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of expression for use in the present invention. H3K9-histone methyltransferase Suv39h1 or HP1α gene expression can be reduced by contacting a subject or cell with a small double stranded RNA (dsRNA), or a vector or construct causing the production of a small double stranded RNA, such that H3K9-histone methyltransferase Suv39h1 or HP1α gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836). All or part of the phosphodiester bonds of the siRNAs of the invention are advantageously protected. This protection is generally implemented via the chemical route using methods that are known by art. The phosphodiester bonds can be protected, for example, by a thiol or amine functional group or by a phenyl group. The 5'- and/or 3'- ends of the siRNAs of the invention are also advantageously protected, for example, using the technique described above for protecting the phosphodiester bonds. The siRNAs sequences advantageously comprises at least twelve contiguous dinucleotides or their derivatives.

As used herein, the term "siRNA derivatives" with respect to the present nucleic acid sequences refers to a nucleic acid having a percentage of identity of at least 90% with erythropoietin or fragment thereof, preferably of at least 95%, as an example of at least 98%, and more preferably of at least 98%.

As used herein, "percentage of identity" between two nucleic acid sequences, means the percentage of identical nucleic acid, between the two sequences to be compared, obtained with the best alignment of said sequences, this percentage being purely statistical and the differences between these two sequences being randomly spread over the nucleic acid acids sequences. As used herein, " best alignment" or "optimal alignment", means the alignment for which the determined percentage of identity (see below) is the highest. Sequences comparison between two nucleic acids sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developped by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p: 1792, 2004). To get the best local alignment, one can preferably used BLAST software. The identity percentage between two sequences of nucleic acids is determined by comparing these two sequences optimally aligned, the nucleic acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.
shRNAs (short hairpin RNA) can also function as inhibitors of expression for use in the present invention.

Ribozymes can also function as inhibitors of expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of H3K9-histone methyltransferase Suv39h1 or HP1α mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable.

Both antisense oligonucleotides and ribozymes useful as inhibitors of expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides, siRNAs, shRNAs and ribozymes of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and preferably cells expressing H3K9-histone methyltransferase Suv39h1 or HP1α. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms (Wu, Z Mol Ther 2006; 14:316-27). Recombinant AAV are derived from the dependent parvovirus AAV2 (Choi, VW J Virol 2005; 79:6801-07). The adeno-associated virus type 1 to 12 can be engineered to be replication deficient and is capable of infecting a wide range of cell types and species (Wu, Z Mol Ther 2006; 14:316-27). It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In a preferred embodiment, the antisense oligonucleotide, siRNA, shRNA or ribozyme nucleic acid sequence is under the control of a heterologous regulatory region, e.g., a heterologous promoter. The promoter may be specific for Muller glial cells, microglia cells, endothelial cells, pericyte cells and astrocytes For example, a specific expression in Muller glial cells may be obtained through the promoter of the glutamine synthetase gene is suitable. The promoter can also be, e.g., a viral promoter, such as CMV promoter or any synthetic promoters.

In a particular embodiment, the inhibitor of H3K9-histone methyltransferase Suv39h1 gene expression is not triptolide.

Inhibitors of the binding of H3K9me3 to HP1α may be selected from small organic molecules, aptamers or polypeptides. In a particular embodiment the polypeptide may be a functional equivalent of HP1α. As used herein, a "functional equivalent of HP1α is a compound which is capable of binding to H3K9me3, thereby preventing the binding of H3K9me3 to HP1α. The term "functional equivalent" includes fragments, mutants, and muteins of HP1α. The term "functionally equivalent" thus includes any equivalent of HP1α obtained by altering the amino acid sequence, for example by one or more amino acid deletions, substitutions or additions such that the protein analogue retains the ability to bind to H3K9me3 but loses the ability to lock the expression of Th1 genes. Amino acid substitutions may be made, for example, by point mutation of the DNA encoding the amino acid sequence. In particular, the functional equivalent comprises all or a portion of HP1α that interacts with H3K9me3. The Polypeptides of the invention may be produced by any suitable means, as will be apparent to those of skill in the art. In order to produce sufficient amounts of the functional equivalent for use in accordance with the present invention, expression may conveniently be achieved by culturing under appropriate conditions recombinant host cells containing the polypeptide of the invention. Preferably, the polypeptide is produced by recombinant means, by expression from an encoding nucleic acid molecule. Systems for cloning and expression of a polypeptide in a variety of different host cells are well known.

As used herein, the term "subject" denotes a mammal, such as a rodent, a feline, a canine, and a primate. Preferably, a subject according to the invention is a human.

In its broadest meaning, the term "treating" or "treatment" refers to reversing, alleviating, inhibiting the progress of, or preventing the disorder or condition to which such term applies, or one or more symptoms of such disorder or condition.

As used herein the term "T-helper type 2 (Th2)-mediated disease" means a disease which is characterized by the overproduction of Th2 cytokines, including those that result from an overproduction or bias in the differentiation of T-cells into the Th2 subtype. Such diseases include, for example, exacerbation of infection with infectious diseases (e.g., Leishmania major, Mycobacterium leprae, Candida albicans, Toxoplasma gondi, respiratory syncytial virus, human immunodeficiency virus, etc.) and allergic disorders, such as anaphylactic hypersensitivity, asthma, allergic rhinitis, atopic dermatitis, vernal conjunctivitis, eczema, urticaria and food allergies, etc. More particularly, Th2-mediated diseases include but are not limited to graft immune diseases (chronic GVHD), autoimmune diseases (especially organ non-specific autoimmune diseases) and type-Th2 allergic diseases. Diseases exemplified typically are ulcerative colitis, systemic lupus erythematodes, myasthenia gravis, systemic progressive scleroderma, rheumatoid arthritis, interstitial cystitis, Hashimoto's diseases, Basedow's diseases, autoimmune hemolytic anemia, idiopathic thrombocytopenic purpura, Goodpasture's syndrome, atrophic gastritis, pernicious anemia, Addison diseases, pemphigus, pemphigoid, lenticular uveitis, sympathetic ophthalmia, primary biliary cirrhosis, active chronic hepatitis, Sjogren's syndrome, multiple myositis, dermatomyositis, polyarteritis nodosa, rheumatic fever, glomerular nephritis (lupus nephritis, IgA nephtopathy, and the like), allergic encephalitis, atopic allergic diseases (for example, bronchial asthma, allergic rhinitis, allergic dermatitis, allergic conjunctivitis, pollinosis, urticaria, food allergy and the like), Omenn's syndrome, vernal conjunctivitis and hypereosinophilic syndrome.

In a particular embodiment, the inhibitors of Suv39h1-HP1α silencing pathway according to the invention are used for the treatment of asthma, in particular allergic asthma.

A further object of the invention relates to a method for the treatment of a T-helper type 2 (Th2)-mediated disease comprising administering a subject in need thereof with an inhibitor of Suv39h1-HP1α silencing pathway of the invention.

Preferably, the inhibitor of Suv39h1-HP1α silencing pathway in a therapeutically effective amount.

By a "therapeutically effective amount" is meant a sufficient amount of the inhibitor of Suv39h1-HP1α silencing pathway to treat a T-helper type 2 (Th2)-mediated disease at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

Inhibitors of Suv39h1-HP1α silencing pathway of the invention may be administered in the form of a pharmaceutical composition, as defined below. Typically, the Inhibitors of Suv39h1-HP1α silencing pathway of the invention can be formulated into pharmaceutical compositions that further comprise a pharmaceutically acceptable carrier, diluent, adjuvant or vehicle. In one embodiment, the present invention relates to a pharmaceutical composition comprising an inhibitor of Suv39h1-HP1α silencing pathway of the invention described above, and a pharmaceutically acceptable carrier, diluent, adjuvant or vehicle. In one embodiment, the present invention is a pharmaceutical composition comprising an effective amount of an inhibitor of Suv39h1-HP1α silencing pathway of the invention or a pharmaceutically acceptable salt thereof and a pharmaceutically acceptable carrier, diluent, adjuvant or vehicle. Pharmaceutically acceptable carriers include, for example, pharmaceutical diluents, excipients or carriers suitably selected with respect to the intended form of administration, and consistent with conventional pharmaceutical practices.

A pharmaceutically acceptable carrier may contain inert ingredients which do not unduly inhibit the biological activity of the compounds. The pharmaceutically acceptable carriers should be biocompatible, e.g., non-toxic, non-inflammatory, non-immunogenic or devoid of other undesired reactions or side-effects upon the administration to a subject. Standard pharmaceutical formulation techniques can be employed.

The pharmaceutically acceptable carrier, adjuvant, or vehicle, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Except insofar as any conventional carrier medium is incompatible with the compounds described herein, such as by producing any undesirable biological effect or otherwise interacting in a deleterious manner with any other component(s) of the pharmaceutically acceptable composition, its use is contemplated to be within the scope of this invention.

Some examples of materials which can serve as pharmaceutically acceptable carriers include, but are not limited to, ion exchangers, alumina, aluminum stearate, lecithin, serum proteins (such as human serum albumin), buffer substances (such as twin 80, phosphates, glycine, sorbic acid, or potassium sorbate), partial glyceride mixtures of saturated vegetable fatty acids, water, salts or electrolytes (such as protamine sulfate, disodium hydrogen phosphate, potassium hydrogen phosphate, sodium chloride, or zinc salts), colloidal silica, magnesium trisilicate, polyvinyl pyrrolidone, polyacrylates, waxes, polyethylene-polyoxypropylene-block polymers, methylcellulose, hydroxypropyl methylcellulose, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The compositions described herein may be administered orally, parenterally, by inhalation spray, topically, rectally, nasally, buccally, vaginally or via an implanted reservoir depending on the severity of the disease being treated.. The term "parenteral" as used herein includes, but is not limited to, subcutaneous, intravenous, intramuscular, intra-articular, intra-synovial, intrasternal, intrathecal, intrahepatic, intralesional and intracranial injection or infusion techniques. Specifically, the compositions are administered orally, intraperitoneally or intravenously.

Sterile injectable forms of the compositions described herein may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions described herein may be orally administered in any orally acceptable dosage form including, but not limited to, capsules, tablets, aqueous suspensions or solutions. In the case of tablets for oral use, carriers commonly used include, but are not limited to, lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions described herein may be administered in the form of suppositories for rectal administration. These can be prepared by mixing the agent with a suitable non-irritating excipient which is solid at room temperature but liquid at rectal temperature and therefore will melt in the rectum to release the drug. Such materials include, but are not limited to, cocoa butter, beeswax and polyethylene glycols.

The pharmaceutical compositions described herein may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

For ophthalmic use, the pharmaceutical compositions may be formulated as micronized suspensions in isotonic, pH adjusted sterile saline, or, specifically, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions may also be administered to the respiratory tract. The respiratory tract includes the upper airways, including the oropharynx and larynx, followed by the lower airways, which include the trachea followed by bifurcations into the bronchi and bronchioli. Pulmonary delivery compositions can be delivered by inhalation by the patient of a dispersion so that the active ingredient within the dispersion can reach the lung where it can, for example, be readily absorbed through the alveolar region directly into blood circulation. Pulmonary delivery can be achieved by different approaches, including the use of nebulized, aerosolized, micellular and dry powder-based formulations; administration by inhalation may be oral and/or nasal. Delivery can be achieved with liquid nebulizers, aerosol-based inhalers, and dry powder dispersion devices. Metered-dose devices are preferred. One of the benefits of using an atomizer or inhaler is that the potential for contamination is minimized because the devices are self contained. Dry powder dispersion devices, for example, deliver drugs that may be readily formulated as dry powders. A pharmaceutical composition of the invention may be stably stored as lyophilized or spraydried powders by itself or in combination with suitable powder carriers. The delivery of a pharmaceutical composition of the invention for inhalation can be mediated by a dosing timing element which can include a timer, a dose counter, time measuring device, or a time indicator which when incorporated into the device enables dose tracking, compliance monitoring, and/or dose triggering to a patient during administration of the aerosol medicament. Examples of pharmaceutical devices for aerosol delivery include metered dose inhalers (MDIs), dry powder inhalers (DPIs), and air-jet nebulizers.

The compounds for use in the methods of the invention can be formulated in unit dosage form. The term "unit dosage form" refers to physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily dose or one of multiple daily doses (e.g., about 1 to 4 or more times per day). When multiple daily doses are used, the unit dosage form can be the same or different for each dose.

A further object of the invention relates to a method for screening a dug for the treatment of a Th2-mediated disease comprising the steps consisting of testing a plurality of test substances for their ability to inhibit the Suv39h1-HP1α silencing pathway and selecting the substances capable of inhibiting said pathway.

In a particular embodiment, the screening method of the invention may comprise the steps consisting of testing a plurality of test substances for their ability to inhibit the methylation of Lys-9 of histone H3 by H3K9-histone methyltransferase Suv39h1 and selecting positively the substances capable of inhibiting the methylation of Lys-9 of histone H3 by H3K9-histone methyltransferase Suv39h1.

Determining whether a test substance is able to inhibit the methylation of Lys-9 of histone H3 by H3K9-histone methyltransferase Suv39h1 may be performed using various methods as described in Greiner D. Et al. Nat Chem Biol. 2005 Aug;1(3):143-5 or Eskeland, R. et al. Biochemistry 43, 3740-3749 (2004). The methods may be performed using high throughput screening techniques for identifying test substances for developing drugs that may be useful to the treatment of a Th2-mediated disease. High throughput screening techniques may be carried out using multi-well plates (e.g., 96-, 389-, or 1536-well plates), in order to carry out multiple assays using an automated robotic system. Thus, large libraries of test substances may be assayed in a highly efficient manner.

In a particular embodiment, the screening method of the invention may comprise the step consisting of a) determining the ability of a plurality of test substances to inhibit the interaction between HP1α and H3K9me3 and b) selecting positively the test substance that inhibit said interaction.

Any method suitable for the screening of protein-protein interactions is suitable. Typically, the polypeptides are labeled with a detectable molecule. Said detectable molecule may consist of any compound or substance that is detectable by spectroscopic, photochemical, biochemical, immunochemical or chemical means. Various assays are described for exploring protein-protein interaction. For example a two hybrid assay may be performed. Alternatively a fluorescence assay performed such as Homogeneous Time Resolved Fluorescence (HTRF) assay, may be performed.

According to a one embodiment of the invention, the test substance of may be selected from the group consisting of peptides, peptidomimetics, small organic molecules, antibodies, aptamers or nucleic acids. For example the test substance according to the invention may be selected from a library of compounds previously synthesized, or a library of compounds for which the structure is determined in a database, or from a library of compounds that have been synthesized de novo.

The substances selected as inhibiting the Suv39h1-HP1α silencing pathway may be then tested for their ability to polarize differentiated Th2 cells into Th1 cells when said Th2 cells are cultured in Th1 conditions. Typically, said ability may tested according the method as described in the EXAMPLE.

The invention will be further illustrated by the following figures and examples. However, these examples and figures should not be interpreted in any way as limiting the scope of the present invention.

### FIGURES:

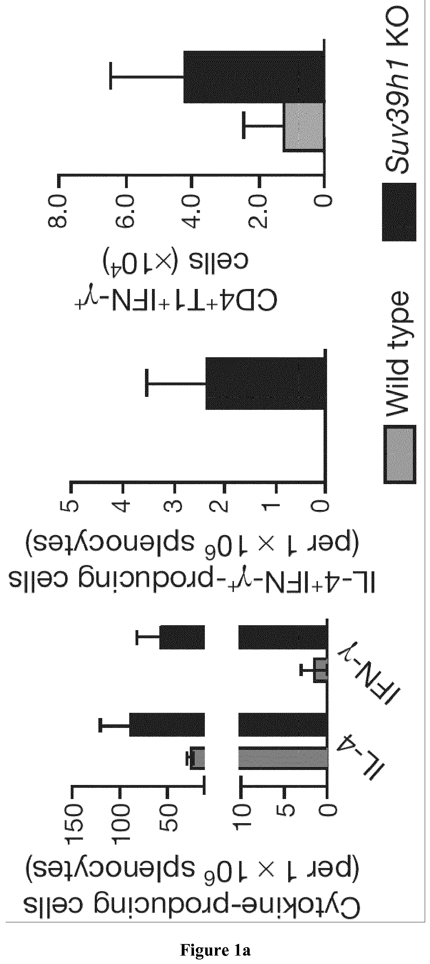

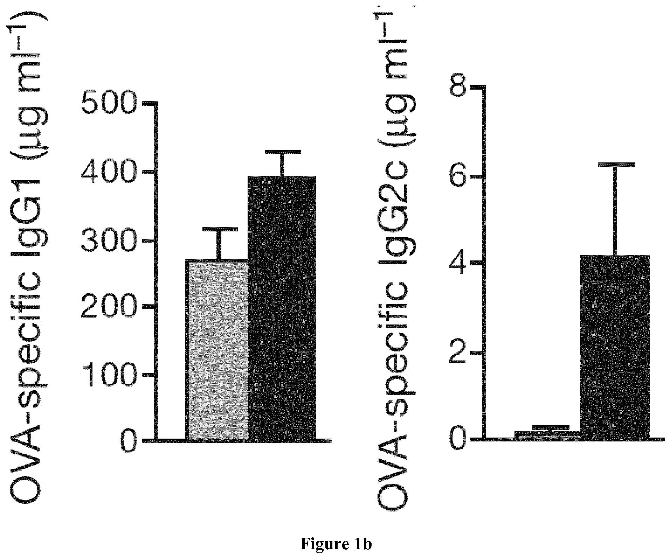

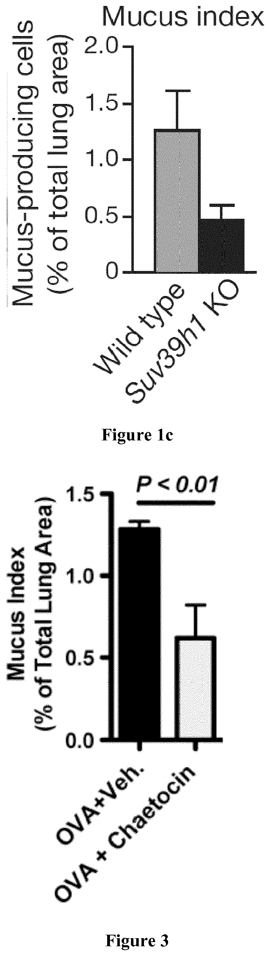

**Figure 1****. Suv39hl regulates Th2 stability in vivo and influences allergic lung inflammation:** Wild type or Suv39h1-deficient mice were injected intraperitoneally on days 0 and 7 with OVA/Alum. On days 55 thru 60 challenged with OVA intranasally and sacrificed on day 61 for analysis. (a) IL4 or IFNγ producing OVA-specific splenocytes were enumerated using dual color ELISpot (left and middle panels). From the same animals the total number of CD4⁺ splenocytes expressing T1/ST2 and producing IFNγ were enumerated by flow cytometry (right panel). (b) The serum levels of OVA-specific IgG1 (left panel) or IgG2c (right panel) were quantified using ELISA. PBS immunized control values were subtracted as background from (a) and (b). (c) Periodic acid Schiff staining was used to quantify the number of mucus positive cells in the lung of OVA challenged animals. All data shown is a representative of two independent experiments with 6 mice per group and is represented as mean±s.e.m.
**Figure 2. Chaetocin treatment results in less allergen-induced lung pathology.** Mice were injected intraperitoneally on days 0 and 7 with OVA/Alum. On day 17 mice were challenged intranasally with OVA and DMSO (OVA+Veh.) or Chaetocin (OVA+Chaetocin) and sacrificed on day 22 for analysis. Periodic acid Schiff staining was used to quantify the number of mucus positive cells (pink staining) in the lung of OVA challenged animals. Images are 200x magnification. All data shown is a pool of two independent experiments with 6 mice per group and is represented as mean±s.e.m.

### EXAMPLE 1: SUV39H1 AND HP1A CONTROL THE FIDELITY OF THE TH2 CELL LINEAGE

### Material & Methods

**Mice:** C57BL/6 were obtained from Charles River (Les Oncins, France) and housed in the animal facility of Institut Curie. We maintained Suv39h1 knockout mice, a kind gift from T. Jenuwein,¹² on a mixed 129SVxC57BL/6 background. The HPla and HP1γ mutant mouse lines were established at the MCI/ICS (Mouse Clinical Institute - Institut Clinique de la Souris-, Illkirch, France; http://www-mci.u-strasbg.fr) and maintained on a mixed 129SVxC57BL/6 background. The details of the strategy are available upon request (project IR00001073/K316). The HPla targeting vector comprises 1) 3.9kb of 5 homology arm in intron 3, 2) a floxed fragment of 1.3kb comprising a LoxP site, 156bp of intron 3, exon 3 and 1030bp of intron 4 and a floxed neo-resistance cassette also surrounded by FRT sites and 3) a 3.4kb of 3' homology arm of intron 4. This construct was electroporated in ES cells (MCI-129sv/Pas) and 733 G-418 resistant clones were screened by PCR with 5' and 3' external primers and a LoxP specific pair of primers. One positive clone for homologous recombination and with only one insertion was isolated and confirmed by Southern blot analysis with a 5' external probe and two Neo specific probes. The karyotype of this ES clone was verified to be normal and this clone was injected in wild-type mice (C57B1/6J). Offsprings were screened by PCR for germ line transmission. Positive mice were then crossed with CMV-Cre transgenic mice to excise the floxed sequence and backcrossed to wild type to obtain HP1α-/- mice. All control wild type mice used were sex-matched littermate controls.

**CD4⁺ T cell purification:** Single cells suspensions of spleens and lymph nodes (mesenteric, inguinal, axillary and brachial) were pooled and after red blood cell lysis CD4⁺T cells purified by negative selection using Miltenyi CD4⁺ T cell isolation kit (Miltenyi Biotec). To obtain naive CD4⁺ T cells, these cells were further purified either by sorting CD44^{low} CD8^{neg} cells using a FACSAria (BD) or FacsVantage (BD) or by positive selection using CD62Lmicrobeads (Miltenyi Biotec). The isolation procedure was performed using 1xPBS (Gibco) containing 0.5% BSA and 2mM EDTA. Cultures obtained from each sorting procedure yielded similar results.

**CD4⁺ T cell cultures:** tissue culture-treated 96 well flat bottom plates (Techno Plastic Products) were coated with 1mg/ml of anti-CD3e (clone 145-2C11,BD) and anti-CD28 (clone 37.51, BD) in 1x PBS (Gibco) for 1-3 hours at 37°C 5% CO₂. Wells were then washed twice with 1xPBS. For non-biased culture conditions wells were seeded with 1x10⁶/ml CD4⁺ T cells in RPMI + Glutamax (Gibco) containing 10% FCS (Gibco), 0.1 mM 2-mercaptoethanol and penicillin and streptomycin. For Th1 culture conditions the medium contained 5ng/ml of recombinant mouse IL12 (R&D systems) and 10mg/ml of anti-IL4 (clone 11B.11, BD or eBiosciences). For Th2 culture conditions the medium contained 50ng/ml of recombinant mouse IL4 (R&D systems) and 10mg/ml of anti-IL12 (C17.8, BD or eBiosciences) and anti-IFNg (clone XMG1.2, BD or eBiosciences). Cells were cultured at 37°C in 5% CO₂. After three days the cells were transferred to fresh plates and the medium was supplemented with a 2x cocktail of the above reagents with the addition of 30U/ml of recombinant human IL-2 (Chiron). If necessary cell populations were expanded every 3 days in medium plus cytokines as described above. For secondary cultures, primary cultures were counted and 1x10⁶/ml were seeded into 96 well plates in the conditions described above.

**Cell staining for flow cytometric analysis:** All antibodies used were purchased from BD with the exception of anti-T-bet (4B10) and anti-Gata3 (HG3-31) which were purchased from Santa Cruz Biotechnology. All staining was done in round-bottom 96 well plates (Corning Life Sciences). Cell Surface staining was performed for 25 minutes at 4°C in 1xPBS 0.5% BSA and 2mM EDTA. For intracellular cytokine staining cells were restimulated at 37°C with PMA (25ng/ml, Sigma) and ionomycin (ImM, Sigma) for 4 hours with the addition of Brefeldin A (5mg/ml) for the last 2 hours. Cell surface staining was performed and then cells were fixed with 2% formaldehyde for 10 minutes at room temperature. The cells were then washed in 1xPBS and then permeabilized by resuspension in Perm/Wash buffer (BD). Cells were centrifuged and intracellular cytokine staining was performed with anti-IFNg and anti-IL4 in permwash buffer for 25 minutes at 4°C. For intracellular transcription factor staining, cells were fixed and permeabilized with 1xPBS containing 0.2% Triton X-100 for 5 minutes at room temperature. Cells were then washed and staining performed in 1xPBS containing 5% BSA. Secondary anti-mouse alexa 488 or 647 (Molecular Probes) were used to detect T-bet and GATA3 primary antibodies. A mouse IgG1 isotype was used as a control. For some experiments fluorophore conjugated T- bet and GATA3-specific antibodies were used (ebioscience). For CFSE experiments, purified T cells were incubated for 10 min at 37°C in PBS with 2.5 µM CFSE (Molecular Probes). All FACS acquisition was performed on a FACScalibur II (BD) using Cell Quest software. Analysis was performed using FlowJo software (Treestar).

**Western blotting:** Cell pellets were lysed in 1x NuPAGE LDS sample buffer (Invitrogen) and 1x NuPAGE reducing agent (Invitrogen). After 30 min treatment with 25 U benzonase nuclease (Novagen), lysate from 2x10⁵ cells were loaded on NuPAGE Bis/Tris 4-12% gradient gels (Invitrogen), using a 1 × MOPS migration buffer (Invitrogen). After transfer, the membranes were blocked with 5% milk in PBS 0.05% Tween and then incubated with primary antibodies and peroxidase-conjugated secondary antibodies. Bound antibodies were revealed using the SuperSignal West Dura Extended Duration Substrate (Thermo Scientific) according to the manufacturers' directions. The intensity of the bands was quantified by densitometry and was expressed as arbitrary units. Anti-HP1α (Euromedex, 2HP-2G9), anti-HP1γ (Euromedex,2MOD-1G6) and anti-HP1β (Euromedex, 1MOD-1A9).

**Chromatin immunoprecipitation:** For ChIP of wild type cells, CD4⁺ T cells from 4-5 week old female C57BL/6 mice were purified by negative selection as described above. For ChIP of Suv39h1-deficient and littermate cells CD4⁺CD44^{low} cells were prepared as described above. ChIP analysis was carried out essentially as described elsewhere ³⁷. Briefly, cells were fixed for 10 min in 1% formaldehyde (wt/vol) at room temperature. Formaldehyde was quenched by 0.125 M glycine for 5 min. Cells were then washed with cold PBS and lysed. The suspension of nuclei was sonicated to achieve an average 200-500 bp length of genomic DNA fragments. Specific antibodies (H3K9me3 (ab 8898 Abcam), H3K9ac (17-657, Upstate), Histone-H3 (ab1791 Abcam), HP1α (05-689, Upstate) and rabbit or mouse IgG (Upstate) were coupled to Dynal protein A beads (Invitrogen). 5 µg of chromatin from each sample was taken ChIP with anti- H3K9me3, 10 µg and for ChIP with anti-H3K9ac and 15 µg for ChIP with anti-HP1α. DNA was purified after crosslink reversal. Real time PCR was performed in triplicate using the Sybr green detection system (Qiagen). Primer sequences are listed in Supplementary table 1. Chromatin immunoprecipitations were performed at least three times with independently cultured cells, results were expressed as a percentage of input DNA and averaged.

**Allergen-Induced Asthma:** Suv39h1-deficient and wild type littermate controls were injected intraperitoneally on days 0 and 7 with 10µg OVA (Sigma) in PBS mixed with 50µl Imject Alum (Thermo Scientific), Imject Alum alone or PBS alone. On days 55 thru 60 anaesthetized mice were challenged with 50µg OVA in 30µl of PBS or PBS alone intranasally. On day 61 mice were sacrificed for analysis.

**Isolation of Total Lung Cells:** Right lung lobes were excised and cut into small pieces (∼1-2mm²). Pieces were transferred into 5mLs of a freshly prepared collagenase digestion solution containing 1,500U Collagenase Type 2 (Worthington) and 75ul of a 10mg/mL DNase I (Roche) stock solution. Lungs were digested for 1 hour at 37C under constant horizontal shaking. 5mL lung digest was transferred into a 40m sieve and pushed through with a sterile syringe top. Following centrifugation red blood cells were lysed with ACK lysis buffer for 1-2 minutes at room temperature. Total lung cells were washed 2 additional times and counted.

**Lung Fixation and Histology:** Left lung lobe was carefully excised and fixed in 3.7% PFA in PBS overnight. Lobes were moved into 70% ETOH the next day. Lung samples were embedded, cut and stained with haematoxylin and eosin (H&E) for cellular infiltration analysis and Periodic acid-Schiff (PAS) for goblet cell hyperplasia analysis. Images were acquired on an Eclipse 90i Upright microscope at the Nikon Imaging Center at the Institut Curie. Mucus index was calculated using ImageJ software (NIH) on entire lung sections.

**Serum Ig:** Flat bottom 96-well Nunc-Immuno Plates (Thermo Scientific) were coated with 10µg/mL OVA or 1/1000 goat anti-mouse Ig (H+L) in PBS (Southern Biotechnologies, cat#1010-01) overnight at 4C. Coating liquid was discarded and wells were blocked with 150µl PBS + 1%BSA for 2 hours at 37C. After discarding blocking buffer, sample and standards were added in 50L per well overnight at 4C. Standards were purchased from Southern Biotechnologies (Mouse IgG1, clone 15H6, starting dilution 10ng/mL in PBS) and (Mouse IgG2c, clone 6.3, starting dilution 10ng/mL). Serum samples were diluted in PBS 1/100 for IgG2c and 1/1,000 for IgG1. Wells were washed 3x with PBS + 0.01% Tween20. Detection IgG1 (goat anti-mouse IgG1-biotin, 0.5mg/mL) and IgG2c (goat anti-mouse IgG2c-biotin, 0.5mg/mL) both from Southern Biotechnologies and used at 1/5000 in 50µl of PBS for 2 hours at 37C. Wells were washed 3x as before and Strepavidin-HRP was added in 50L of PBS (R&D, 1/200 dilution) for 30 minutes at 37C. After 3 washes, 150L of TMB substrate was added to each well (Sigma). The reaction was stopped by adding 50µl/well of 1N H₂SO₄. Plates were read at 450nm and 570nm, the latter for background wavelength correction.

**ELISpot:** Splenocytes were seeded at 1x10⁶ and 0.5x10⁶ cells per well with and without 50µg/mL OVA for 2 days. Dual IFNg/IL4 ELISpot was performed according to manufacture's protocol (R&D Systems, cat# ELD5217).

### Results

After antigen encounter, CD4⁺ helper T (Th) cell differentiation follows distinct developmental programs that generate different types of effector T lymphocytes, including Th1, Th2, Th17 or Treg. Each subtype is characterized by specific expression of lineage- instructive transcription factors and signature cytokines^{1,2}. Recently, the stability and plasticity of Th phenotypes has been recognized as critical to immune responses^{3,4}.

Differentiation of Th1 and Th2 cells from a common precursor occurs by cross-antagonism between the master-regulators T-bet and GATA-3, respectively, and leads to a mutually exclusive expression of cytokines genes^{1,2,3}. Th1 cells produce IFNγ, which is important for clearance of intracellular pathogens, whereas Th2 cells produce IL-4, IL-5, IL-10 and IL-13 and are critical for humoral immunity and clearance of extracellular pathogens¹.

As Th1 and Th2 phenotypes are heritable through cellular divisions^{5,6,7}, it has been proposed that epigenetic modifications regulate T cell differentiation^{1,3,4,8}. Interestingly, correlations were observed between levels of several histone-H3 and H4 modifications with activity or silencing of cytokine genes in committed Th1 and Th2 cells⁹. Moreover, the combination of active (H3K4me3) and inactive (H3K27me3) marks in the loci of transcription factors was proposed to shape their transcriptional potential¹⁰. However, the actual pathways underlying the establishment of epigenetic marks have not been directly manipulated to demonstrate their implication in T cell differentiation³.

Intriguingly, the role of one important epigenetic mark predominantly associated with transcriptionally inert heterochromatin, trimethylation of the lysine 9 residue of Histone-H3 (H3K9me3), has not been investigated thoroughly during Th cell differentiation. A 2-member family of histone methyltransferases (HMTases), Suv39h1 and Suv39h2^{11,12,13} (also known as KMT1A and KMT1B, respectively¹⁴) are responsible for trimethylation of H3K9 in pericentric heterochromatin. H3K9me3 in turn, can serve as a binding site for a histone binding adaptor family, heterochromatin protein 1 (HP1α, β and γ) ^{15,16,17}. HP1 was originally identified in *Drosophila* and defined based on its property to act as a dominant suppressor of position effect variegation on gene silencing¹⁸. Since then, a number of groups have provided evidence that a HP1 repressive loop can silence transgene expression in mammals^{15,17,19,20}. Current views propose that Suv39h1 trimethylates H3K9 in pericentromeric regions leading to the recruitment of HP1, which either sterically inhibits the binding of transcriptional machinery or attracts diverse chromatin modifiers, allowing maintenance and propagation of heterochromatin²¹. Suv39h1 can also associate with histone deacetylases (HDACs) and other HMTases, suggesting a complex mechanism of Suv39h1-mediated silencing, including maintenance of a hypoacetylated state of H3K9^{22,23}.

In order to investigate a role for this silencing system in Th cell differentiation, we examined the post-translational modifications to H3K9 within the promoters of Th lineage determining genes by chromatin immunoprecipitation (ChIP), using antibodies specific for H3K9me3 (a mark usually associated with silencing) and H3K9ac (a typical mark of active genes). Importantly, we screened for batches of commercial antibodies that did not cross-react with other histone marks and identified one batch of anti-H3K9me3 with low cross reactivity to all other marks tested. Anti-H3K9me3 effectively precipitated major satellite sequences from pericentric heterochromatin, where this mark is enriched, and did not precipitate the promoter region of active Gapdh gene. On the contrary, H3K9ac was associated with the promoter of the active Gapdh gene, but not with major satellites.

During *in vitro* CD4⁺ Th differentiation we observed by day 7 an increase ofrepressive H3K9me3 in the promoters of lineage-inappropriate cytokine genes (114 in Th1 and Ifng in Th2) and an increase of H3K9ac at the promoters of lineage-specifying cytokine genes. The promoter of Ifng initially contained higher levels of H3K9me3 in naive CD4⁺ T cells than that of IL4. During lineage commitment, we observed a 2.5 fold increase in repressive H3K9me3 in the promoter of silent Ifng (in Th2 cells) and a four-fold increase in the promoter of silent IL4 (in Th1 cells). Similarly, a five-fold enrichment of H3K9ac was observed in the promoter of active Ifng and up to 30-fold in the active 114 promoter.

We then examined H3K9 modifications at the promoters of the genes coding Th1 and Th2 lineage-instructive transcription factors. There was a high level of the repressive H3K9me3 mark at the promoter of Tbx21 (coding for T-bet) in naive cells, yet during Th1 differentiation, H3K9me3 decreased, concomitant with a five-fold increase in H3K9ac. In Th2 cells, the hypermethylated and hypoacetylated status of H3K9 at the Tbx21 promoter was maintained. The promoter of Th2-specifying Gata3 displayed an approximate six-fold increase in H3K9me3 in Th1 cells and two-fold increase in H3K9ac in Th2 cells. Our results show that the balance between trimethylation and acetylation of H3K9 in Th1 and Th2 gene promoters is regulated in a lineage-specific manner, and correlates with gene silencing or activation, respectively.

The observed modifications in H3K9 could, of course, be either a cause or consequence of the differences in gene expression between Th1 and Th2 cells. To address this, we used mice lacking the H3K9 tri-methyltransferase Suv39h1 (in adult tissues Suv39h2 expression if restricted to testes¹¹). As previously reported^{12,24}, these mice developed normally and had the predicted ratio and phenotype of hematopoietic cells in the lymphoid organs. Next we explored a possible role for Suv39h1 in Th cell polarization. There was little difference between wild type (age and sex-matched littermate controls) and Suv39h1-deficient cells in up-regulation of surface activation markers or cell proliferation in Th1 or Th2 culture conditions. We then examined the differentiation of CD4⁺ T cells for 7 days in non-biased (NB), Th1 and Th2 conditions by assessing intracellular cytokine accumulation and expression of Th1 and Th2 master regulators, Tbet and Gata3, respectively. We did not find any major differences between the wild type littermate cells and Suv39h1-deficient cells. Thus, Suv39h1-deficient cells polarize normally *in vitro,* suggesting that H3K9 trimethylation is not involved in the silencing of the opposite loci during differentiation. However, these cells were polarized in highly biased conditions where they were incubated with the optimal concentrations of the polarizing cytokines and antibodies neutralizing the opposite cytokines. We therefore reasoned that incubating the already differentiated Th1 or Th2 cells under the opposite polarizing conditions may reveal potential defects in silencing of the opposite loci.

To test this possibility, differentiated Th1 or Th2 cells (day 7 of the primary cultures) were re-stimulated for 2-3 days under the opposite polarizing conditions (Th1 conditions for Th2 cells, and Th2 conditions for Th1). In wild type Th2 cells, we observed only low induction of the silenced Th1 or Th2 genes by Th1 cells in the secondary cultures, indicating that the silencing of the opposite lineage genes during Th1/Th2 differentiation is irreversible under these conditions. Similarly, Suv39h1-deficient Th1 cells did not express IL4 or GATA3 in secondary Th2 cultures. In contrast, upon exposure of Suv39h1-deficient Th2 cells to secondary Th1 conditions, the silenced IFNγ and T-bet were both induced in a significant proportion (∼30%) of the cells.

The increase in plasticity observed in the Suv39h1-deficient Th2 cells when cultured in Th1 conditions could be due to a silencing defect in fully differentiated Th2 cells, or to uncommitted cells (Gata-3/IL4-negative) that persisted in the Th2 cultures and induced expression of IFNγ and T-bet under Th1 conditions. To address this question, we gated on Th2 cells that maintained expression of IL4 or GATA3 after secondary Th1 re-stimulation and examined their levels of IFNγ or T-bet. The proportion of IL4 and GATA3-positive Th2 cells that co-express IFNγ and T-bet in the secondary Th1 cultures was increased in the Suv39h1-deficient cells. Therefore, in the absence of Suv39h1, increased levels IFNγ and T-bet are due to a true plasticity of differentiated IL4⁺/GATA3⁺ Th2 cells, and not to undifferentiated T cells in the Th2 cultures. We conclude that Suv39h1 restricts Th2 plasticity by locking out the Th1 lineage genes in differentiated Th2 cells.

In order to address the mechanisms involved in the stable silencing of the Th1 lineage genes IFNγ and T-bet, we analyzed wild type and Suv39h1-deficient Th2 cells by ChIP, using antibodies against both H3K9me3 and H3K9ac. Importantly, the Suv39h1-deficient Th2 cell populations were indistinguishable from wild type Th2 cells in terms of IL4/GATA3 and IFNγ/T-bet expression. Both populations were also homogenous, as GATA3 was expressed in virtually 100% of the cells. As expected, ChIP analysis for major satellites showed a three-fold decrease of H3K9me3, in comparison to wild type littermates, while no H3K9ac was detected. Opposite results were obtained with ChIP for the active Gadph gene: no H3K9m3 signal and a strong H3K9ac signal, which was unchanged in Suv39h1-deficient cells.

As expected from our analysis, the patterns of methylation and acetylation reflected Th2 polarization (i.e. higher levels of H3K9me3 in silenced genes and of H3K9ac in active genes). In Suv39h1-deficient Th2 cells, H3K9me3 levels in the promoter of Tbx21 persisted, while a reproducible increase in acetylation was also observed here. In wild type Th2 cells, repressive H3K9me3 is non-uniformly spread across regulatory elements (so-called conserved non-coding regions, CNS) encompassing 5' and 3' gene flanking sequences, which are indispensable for proper expression of IFNγ¹. Suv39h1- deficient Th2 cells showed specific decreases in H3K9me3 levels in several, but not all regulatory elements. Significant decrease in densities of H3K9me3 was observed in the promoter of Ifng and proximal CNS (+54kb), and stronger decrease at CNS (-53kb) and proximal CNS (-6kb). At the same time, we observed hyperacetylation of H3K9 within the promoter of Ifng and CNS regions from which H3K9me3 was decreased. Of note, ChIP with the antibodies for histone H3 did not show any significant changes in nucleosomal density at the regions of interest in Suv39h1-deficient cells. The imbalance between repressive and active H3K9 modifications in the Suv39h1-deficient Th2 cells was restricted to the Ifng and Tbx21 gene promoters, while other Th1-related transcription factors, such as Stat1 and Stat4, as well as Th2-related Stat6 did not show major alterations. In Suv39h1-deficient cells there was no increase in H3K9ac at promoters of Th2-specifying IL4 and Gata3. Therefore, in Th2 cells, Suv39h1 deficiency causes an imbalance between H3K9me3 and H3K9ac at the Th1 gene loci, resulting in incomplete silencing and increased Th2-to-Th1 plasticity. As Suv39h1 is known to associate with HDACs²², the observed increase of H3K9ac in Suv39h1 gene targets in Th2 cells might reflect the loss of this silencing component. These results suggest that the incomplete silencing of the Th1 genes (Ifng and Tbx21) in Suv39h1-deficient Th2 cells is the consequence of the observed imbalance of the H3K9me3/H3K9ac ratio in the corresponding gene promoters.

Given the known connection between Suv39h1 and HP1 in heterochromatin²¹, we investigated the potential involvement of HP1 in Th1 gene silencing in Th2 cells by generating mice deficient in HPla by targeted deletion. These mice developed normally, and had the predicted ratio and phenotype of hematopoietic cells in the lymphoid organs. When stimulated, CD4⁺ T cells from HP1α-deficient mice displayed comparable up-regulation of cell surface activation markers and cell division profiles to wild type. After seven days of differentiation in polarizing in Th1 or Th2 conditions, we detected no difference in intracellular cytokine profiles or T-bet and GATA3 expression levels between the HP1α-deficient and wild type cells. We conclude that, similar to Suv39h1-deficient cells, CD4⁺ T cells deficient for HP1α have no defects in activation, proliferation or Th1/Th2 polarization.

We then investigated the putative role for HPla in Th2-plasticity by re-culturing HP1α-deficient Th1 or Th2 cells under the opposite polarization conditions, as before. We observed a marked increase in IFNγ production by HP1α-deficient Th2 cells compared to wild type cells, similar to that observed in cells lacking Suv39h1. There were also a similar proportion of IL4⁺ cells from HP1α-deficient mice that expressed IFNγ in these secondary cultures (∼22%). HP1α-deficient Th1 cells behaved as their wild type counterparts in the corresponding opposite experiment. We also observed greater induction of T-bet in the secondary Th1 cultures of HP1α-deficient Th2 cells, as compared to wild type. Thus, HP1α is involved in the maintenance of Th1 gene silencing in Th2 cells.

Due to the high degree of homology between the three HP1-isoforms, we tested if the effects observed were specific to HPla. To do this, we used cells from HP1γ deficient mice (HP1β-deficiency is lethal in mice²⁵). We did not see any difference in the production of IFNγ between HP1γ-deficient and wild type Th2 cells, showing that reversibility of silencing of Th1 gene expression in Th2 cells is specific to HP1α. Therefore, HPla is required for the effective silencing of the Th1 gene loci in Th2 cells and the restriction of Th2-to-Th1 plasticity, possibly via Suv39h1.

We tested this possibility directly by measuring the recruitment of HPla to the Th1 promoter in both wild type and Suv39h1-deficient Th2 cells. HP1α was indeed bound to the silenced Ifng and Tbx21 promoters in wild type Th2 cells. However, in Suv39h1-deficient Th2 cells this binding was markedly reduced, showing that the recruitment of HP1α to Th1 gene promoters in Th2 cells is Suv39h1-dependent. We conclude that during Th2 lineage commitment, silencing by the Suv39h1/HP1α pathway lock out the Th1-specifying genes, thereby restricting Th2-to-Th1 plasticity.

Finally, we reasoned that if the defective silencing of the Ifng and Tbx21 loci in the Suv39h1-deficient Th2 cells compromised lineage commitment and increased plasticity, we would observe a shift toward a Th1 responses *in vivo.* We investigated this possibility in a model of ovalbumin (OVA)-induced allergic asthma that promotes a strict Th2-type response resulting in allergen-induced lung pathology²⁶. As expected, immunological sensitization of wild type mice to OVA resulted in the generation of an antigen-specific Th2 response characterized by OVA-specific production of IL4 and low levels of IFNγ in the spleen (Fig. 1a). In addition to the generation of IL4⁺ T cells, in Suv39h1-deficient mice we also observed abundant allergen-specific IFNγ⁺ cells and IL4/IFNy double producers, indicative of Th2 instability (Fig. 1a). Suv39h1-deficient mice also had increased numbers of CD4⁺ T cells that expressed the Th2 marker T1/ST2 and produced IFNγ (Fig. 1a; right panel). Evidence of an increased Th1 response in the Suv39h1-deficient mice was further supported by the different IgG isotypes in the serum. In wild type mice, as expected, we found high levels of OVA- specific IgG1 (a surrogate marker for the induction of Th2 responses), and barely detectable levels of the Th1-induced OVA-specific IgG2c²⁷ in the serum (Fig. 1b). In line with a response skewed toward Th1, serum from the Suv39h1 KO mice contained increased levels of IgG2c when compared to their wild type counterparts (Fig. 1b). Therefore, consistent with our *in vitro* results, Suv39h1-deficient mice show defective Th2-lineage stability *in vivo.*

To determine whether the increase in Th2 plasticity had a pathophysiological consequence, we evaluated lung inflammation in the immunized mice. Indicative of strong Th2-mediated lung disease²⁸, OVA-immunization of wild type mice resulted in an intense eosinophil infiltration and mucus production in the lungs (Fig. 1c). In Suv39h1-deficient mice, we observed a marked reduction in both eosinophil infiltration and mucus production. Similar results were also obtained in bone-marrow irradiation chimeras, in which the lung epithelium expressed Suv39h1, indicating that the differences between wild type and Suv39h1-deficient mice are restricted to the hematopoietic compartmen. We found that increased Th2 plasticity caused skewed Th1 responses in a strict Th2 model, resulting in reduced allergen-specific inflammatory response (as suggested before by otherS^{29,30,31}). Taken together, these results indicate that Suv39h1-silencing controls the stability of Th2 cell identity *in vivo.*

In conclusion, we have identified an epigenetic pathway responsible for maintaining the phenotypic stability of Th2 cells by silencing Th1 genes. The Suv39h1-HPla loop has been long associated with constitutive pericentric heterochromatin where it maintains a stable, silent environment^{17,32}. However, whether this is restricted to pericentric heterochromatin has remained unexplored. Here we show that these factors could contribute to the regulation of haematopoietic genes, such as the Ifng and Tbx21. We suggest a model in which the loss of Suv39h1 in Th2 cells leads to the perturbation of the homeostasis in H3K9 post- translational modifications in silenced Th1-loci. This decrease of repressive H3K9me3 concomitant with the acquisition of the active H3K9ac mark has no consequences in normal polarizing culture conditions. However, under *in vivo* conditions, this imbalance leads to a loss of Th2 stability and an acquisition of a chimeric Th2+Th1 phenotype. This is potentially due to both the increased permissiveness of Ifng locus for Tbet and a repulsion of HP1α repressive element by increased levels of H3K9ac as has been observed previously³³. Given that we observed a similar phenotype with HP1α-deficient cells, we propose that HP1α serves as a Suv39h1/H3K9me3-dependant molecular lock. Suv39h-mediated silencing mechanisms are known to involve, in addition to HP1α, diverse co-repressors such as KAP1 and DNA methyltransferases (DNMT) and HDACs, which can directly modify chromatin ^{19,22,34,35,36} . Indeed in the increase in H3K9ac in Suv39h1 target regions strongly suggests a loss of HDACs. In the case of the Th2 cell lineage, the exact mechanism of the multimeric Suv39h 1/HP1a "locking" module will be the subject of further investigations to elucidate the control of stability and plasticity of Th phenotypes.

### EXAMPLE 2: CHAETOCIN TREATMENT RESULTS IN LESS ALLERGEN-INDUCED LUNG PATHOLOGY.

### Methods:

6-8 week old female C57B1/6 mice were injected intraperitoneally on days 0 and 7 with 10mg OVA (Sigma) in PBS mixed with 50µl Imject Alum (Thermo Scientific). On days 17 to 22 anaesthetized mice were sensitized intranasally with 50mg OVA in 30ul of PBS mixed with 0.25mg/kg of chaetocin (Sigma) or with vehicle (DMSO). On day 22 mic e were sacrificed for analysis.

### Results:

To assess whether allergic asthma pathology could be reduced by the inhibition of Suv39h1 we used a model of OVA induced allergic asthma. Mice were treated as shown in the experimental design above. In this model mice develop allergic responses that results in the production of mucus in the airways. Figure 2 shows that the production of mucus in the airways was significantly greater in mice treated with the allergen (OVA) and the vehicle control that when OVA was administered in conjunction with chaetocin. These results show that inhibition of Suv39h1 in the lung reduces allergic asthma pathology. We conclude that chaeotocin has a potential usage for asthma treatment.

### REFERENCES:

Throughout this application, various references describe the state of the art to which this invention pertains. The disclosures of these references are hereby incorporated by reference into the present disclosure.
1 Wilson, C. B., Rowell, E. & Sekimata, M. Epigenetic control of T-helper-cell differentiation. Nat Rev Immunol 9, 91-105, doi:nri2487 [pii] 10.1038/nri2487 (2009).
2 Zhou, L., Chong, M. M. & Littman, D. R. Plasticity of CD4+ T cell lineage differentiation. Immunity 30, 646-655, doi:S1074-7613(09)00198-8 [pii] 10.1016/j.immuni.2009.05.001 (2009).
3 Murphy, K. M. & Stockinger, B. Effector T cell plasticity: flexibility in the face of changing circumstances. Nat Immunol 11, 674-680, doi:ni.1899 [pii] 10.1038/ni.1899 (2010).
4 O'Shea, J. J. & Paul, W. E. Mechanisms underlying lineage commitment and plasticity of helper CD4+ T cells. Science 327, 1098-1102, doi:327/5969/1098 [pii] 10.1126/science.1178334 (2010).
5 Bird, J. J. et al. Helper T cell differentiation is controlled by the cell cycle. Immunity 9, 229-237, doi:S1074-7613(00)80605-6 [pii] (1998).
6 Grogan, J. L. et al. Early transcription and silencing of cytokine genes underlie polarization of T helper cell subsets. Immunity 14, 205-215, doi:S1074-7613(01)00103-0 [pii] (2001).
7 Richter, A., Lohning, M. & Radbruch, A. Instruction for cytokine expression in T helper lymphocytes in relation to proliferation and cell cycle progression. J Exp Med 190, 1439-1450 (1999).
8 Smale, S. T. The establishment and maintenance of lymphocyte identity through gene silencing. Nat Immunol 4, 607-615, doi:10.1038/ni0703-607 ni0703-607 [pii] (2003).
9 Avni, O. et al. T(H) cell differentiation is accompanied by dynamic changes in histone acetylation of cytokine genes. Nat Immunol 3, 643-651, doi:10.1038/ni808 ni808 [pii] (2002).
10 Wei, G. et al. Global mapping of H3K4me3 and H3K27me3 reveals specificity and plasticity in lineage fate determination of differentiating CD4+ T cells. Immunity 30, 155-167, doi:S1074-7613(08)00555-4 [pii] 10.1016/j.immuni.2008.12.009 (2009).
11 O'Carroll, D. et al. Isolation and characterization of Suv39h2, a second histone H3 methyltransferase gene that displays testis-specific expression. Mol Cell Biol 20, 9423-9433 (2000).
12 Peters, A. H. et al. Loss of the Suv39h histone methyltransferases impairs mammalian heterochromatin and genome stability. Cell 107, 323-337, doi:S0092-8674(01)00542-6 [pii] (2001).
13 Rea, S. et al. Regulation of chromatin structure by site-specific histone H3 methyltransferases. Nature 406, 593-599, doi: 10.1038/35020506 (2000).
14 Allis, C. D. et al. New nomenclature for chromatin-modifying enzymes. Cell 131, 633-636, doi:S0092-8674(07)01359-1 [pii] 10.1016/j.cell.2007.10.039 (2007).
15 Bannister, A. J. et al. Selective recognition of methylated lysine 9 on histone H3 by the HP1 chromo domain. Nature 410, 120-124, doi:10.1038/35065138 35065138 [pii] (2001).
16 Elgin, S. C. & Grewal, S. I. Heterochromatin: silence is golden. Curr Biol 13, R895-898, doi:S0960982203008376 [pii] (2003).
17 Lachner, M., O'Carroll, D., Rea, S., Mechtler, K. & Jenuwein, T. Methylation of histone H3 lysine 9 creates a binding site for HP1 proteins. Nature 410, 116-120, doi:10.1038/35065132 35065132 [pii] (2001).
18 James, T. C. & Elgin, S. C. Identification of a nonhistone chromosomal protein associated with heterochromatin in Drosophila melanogaster and its gene. Mol Cell Biol 6, 3862-3872 (1986).
19 Ayyanathan, K. et al. Regulated recruitment of HP1 to a euchromatic gene induces mitotically heritable, epigenetic gene silencing: a mammalian cell culture model of gene variegation. Genes Dev 17, 1855-1869, doi:10.1101/gad. 1102803 1102803 [pii] (2003).
20 Nielsen, S. J. et al. Rb targets histone H3 methylation and HP1 to promoters. Nature 412, 561-565, doi:10.1038/35087620 35087620 [pii] (2001).
21 Maison, C. & Almouzni, G. HP1 and the dynamics of heterochromatin maintenance. Nat Rev Mol Cell Biol 5, 296-304, doi:10.1038/nrm1355 nrm1355 [pii] (2004).
22 Vaute, O., Nicolas, E., Vandel, L. & Trouche, D. Functional and physical interaction between the histone methyl transferase Suv39H1 and histone deacetylases. Nucleic Acids Res 30, 475-481 (2002).
23 Fritsch, L. et al. A subset of the histone H3 lysine 9 methyltransferases Suv39h1, G9a, GLP, and SETDB1 participate in a multimeric complex. Mol Cell 37, 46-56, doi:S1097-2765(09)00921-6 [pii] 10.1016/j.molcel.2009.12.017 (2010).
24 Braig, M. et al. Oncogene-induced senescence as an initial barrier in lymphoma development. Nature 436, 660-665, doi:nature03841 [pii] 10.1038/nature03841 (2005).
25 Aucott, R. et al. HP1-beta is required for development of the cerebral neocortex and neuromuscular junctions. J Cell Biol 183, 597-606, doi:jcb.200804041 [pii] 10.1083/jcb.200804041 (2008).
26 Kumar, R. K., Herbert, C. & Foster, P. S. The "classical" ovalbumin challenge model of asthma in mice. Curr Drug Targets 9, 485-494 (2008).
27 Snapper, C. M., Peschel, C. & Paul, W. E. IFN-gamma stimulates IgG2a secretion by murine B cells stimulated with bacterial lipopolysaccharide. J Immunol 140, 2121-2127 (1988).
28 Gonzalo, J. A. et al. Eosinophil recruitment to the lung in a murine model of allergic inflammation. The role of T cells, chemokines, and adhesion receptors. J Clin Invest 98, 2332-2345, doi: 10.1172/JCI119045 (1996).
29 Romagnani, S. Immunologic influences on allergy and the TH1/TH2 balance. J Allergy Clin Immunol 113, 395-400, doi:10.1016/j.jaci.2003.11.025 S0091674903026873 [pii] (2004).
30 Barnes, P. J. The cytokine network in asthma and chronic obstructive pulmonary disease. J Clin Invest 118, 3546-3556, doi:10.1172/JCI36130 (2008).
31 Barnes, P. J. Immunology of asthma and chronic obstructive pulmonary disease. Nat Rev Immunol 8, 183-192, doi:nri2254 [pii] 10.1038/nri2254 (2008).
32 Grewal, S. I. & Jia, S. Heterochromatin revisited. Nat Rev Genet 8, 35-46, doi:nrg2008 [pii] 10.1038/nrg2008 (2007).
33 Maison, C. et al. Higher-order structure in pericentric heterochromatin involves a distinct pattern of histone modification and an RNA component. Nat Genet 30, 329-334, doi:10.1038/ng843 ng843 [pii] (2002).
34 Cammas, F., Herzog, M., Lerouge, T., Chambon, P. & Losson, R. Association of the transcriptional corepressor TIFlbeta with heterochromatin protein 1 (HP1): an essential role for progression through differentiation. Genes Dev 18, 2147-2160, doi:10.1101/gad.302904 18/17/2147 [pii] (2004).
35 Fuks, F., Hurd, P. J., Deplus, R. & Kouzarides, T. The DNA methyltransferases associate with HP1 and the SUV39H1 histone methyltransferase. Nucleic Acids Res 31, 2305-2312 (2003).
36 Smallwood, A., Esteve, P.O., Pradhan, S. & Carey, M. Functional cooperation between HP1 and DNMT1 mediates gene silencing. Genes Dev 21, 1169-1178, doi:gad. 1536807 [pii] 10.1101/gad. 1536807 (2007).
37 Lee, T. I., Johnstone, S. E. & Young, R. A. Chromatin immunoprecipitation and microarray-based analysis of protein location. Nat Protoc 1, 729-748, doi:nprot.2006.98 [pii] 10.1038/nprot.2006.98 (2006).

## Claims

1. An inhibitor of the Suv39h1-HP1α silencing pathway for use in the treatment of a T-helper type 2 (Th2)-mediated disease.

2. The inhibitor of the Suv39h1-HP1α silencing pathway for use according to claim 1 which is selected from the group consisting of inhibitors of H3K9-histone methyltransferase Suv39h1; inhibitors of H3K9-histone methyltransferase Suv39h1 gene expression, inhibitors of HP1α gene expression and inhibitors of the binding of H3K9me3 to HP1α.

3. The inhibitor of the Suv39h1-HP1α silencing pathway for use according to claim 1 or 2 which is chaetocin.

4. The inhibitor of the Suv39h1-HP1α silencing pathway for use according to any of the preceding claims wherein said T-helper type 2 (Th2)-mediated disease is selected from the group consisting of graft immune diseases (chronic GVHD), autoimmune diseases (especially organ non-specific autoimmune diseases) and type-Th2 allergic diseases. Diseases exemplified typically are ulcerative colitis, systemic lupus erythematodes, myasthenia gravis, systemic progressive scleroderma, rheumatoid arthritis, interstitial cystitis, Hashimoto's diseases, Basedow's diseases, autoimmune hemolytic anemia, idiopathic thrombocytopenic purpura, Goodpasture's syndrome, atrophic gastritis, pernicious anemia, Addison diseases, pemphigus, pemphigoid, lenticular uveitis, sympathetic ophthalmia, primary biliary cirrhosis, active chronic hepatitis, Sjogren's syndrome, multiple myositis, dermatomyositis, polyarteritis nodosa, rheumatic fever, glomerular nephritis (lupus nephritis, IgA nephtopathy, and the like), allergic encephalitis, atopic allergic diseases (for example, bronchial asthma, allergic rhinitis, allergic dermatitis, allergic conjunctivitis, pollinosis, urticaria, food allergy and the like), Omenn's syndrome, vernal conjunctivitis and hypereosinophilic syndrome.

5. The inhibitor of the Suv39h1-HP1α silencing pathway for use according to any of the preceding claims wherein said T-helper type 2 (Th2)-mediated disease is asthma or allergic asthma.

6. A method for screening a dug for the treatment of a Th2-mediated disease comprising the steps consisting of testing a plurality of test substances for their ability to inhibit the Suv39h1-HP1α silencing pathway and selecting the substances capable of inhibiting said pathway.
